# EUROPÄISCHE PATENTSCHRIFT

(11) **EP 1 038 540 B1**
(45) Veröffentlichungstag und Bekanntmachung des Hinweises auf die Patenterteilung: **24.05.2006**
(21) Anmeldenummer: 00105617.5
(22) Anmeldetag: 16.03.2000
(51) Int. Cl.: A61L 27/18, A61L 31/06, A61L 17/12, D01F 6/62

(54) **Strangförmiges Implantat aus resorbierbarem Polymermaterial, Verfahren zu seiner Herstellung**
Elongated implant made of a bioresorbable polymer and manufacturing method
Implant filiforme en matériau biorésorbable et son procédé de préparation

(30) Priorität: 19.03.1999 DE 19912360
(43) Veröffentlichungstag der Anmeldung: 27.09.2000
(73) Patentinhaber: Aesculap AG & Co. KG, 78532 Tuttlingen (DE)
(72) Erfinder: Dauner, Martin, 73732 Esslingen (DE); Goldmann, Helmut, Dr., 78532 Tuttlingen (DE); Hierlemann, Helmut, Dr., 73033 Göppingen (DE); Planck, Heinrich, Prof. Dr., 72622 Nürtingen (DE)
(74) Vertreter: Patentanwälte Ruff, Wilhelm, Beier, Dauster & Partner

(56) Entgegenhaltungen:
- EP-A- 0 241 252
- WO-A-98/20190
- US-A- 5 383 931
- US-A- 5 425 766

## Beschreibung

Die vorliegende Erfindung betrifft ein strangförmiges Implantat aus resorbierbarem Polymermaterial, ein Verfahren zu seiner Herstellung und seine Anwendung in der Chirurgie.

In der Chirurgie werden Implantate häufig zur Befestigung und Unterstützung sowie als zeitweiliger oder dauernder Ersatz für geschädigte Körperteile eingesetzt. Als Beispiel sind hierbei chirurgische Nähfäden, Gefäßprothesen sowie künstliche Sehnen und Bänder zu nennen.

Es wurden Implantate in Form von Kordeln oder Bändern entwickelt, insbesondere für Anwendungen in der orthopädischen Chirurgie von Menschen und Tieren. Sie werden beispielsweise bei der Rekonstruktion oder Prothetik im Bereich des Bewegungsapparates, wie etwa einem Kreuzbandriß, verwendet.

Jedoch zeigen sich bei Verwendung nichtresorbierbarer Materialien, wie beispielsweise Polytetrafluorethylen oft persistierende Fremdkörperreaktionen sowie durch das Implantat bedingte Spätinfektionen.

Deshalb wurden Entwicklungen auf teilweise oder vollständig im Körper resorbierbare Implantatmaterialien gerichtet. Hierzu ist die von der Firma Ethicon im deutschen Patent DE-C2 4012602 offenbarte Implantatkordel aus Polydioxanon zu nennen. In der EP-B1 0 241 252 ist ein Lactid/Glykolid-Polymer beschrieben, das mit Dodecanol endverkappt ist.

Die aus dem Stand der Technik bekannten Kordeln oder Bänder weisen eine Reihe von Nachteilen auf. So zeigen einige resorbierbare Materialien eine beschleunigte Resorption und damit eine zu schnelle Festigkeitsverringerung, was den Heilungserfolg beeinträchtigt. Hohe Steifigkeit der Kordeln führt zu schlechtem Verknotungsverhalten und Risiken ungenauer Anordnung im Operationsgebiet. Ferner beeinträchtigen rauhe Oberflächen der Kordeln das Durchzugverhalten und können umgebendes Körpergewebe beschädigen.

Die Erfindung stellt sich die Aufgabe, ein strangförmiges Implantat aus einem resorbierbaren Polymer zur Verfügung zu stellen, das gute mechanischen Eigenschaften in Kombination mit einem gutem Abbau- und Resorptionsverhalten in vivo besitzt, das einfach und kostengünstig herzustellen und auch einfach und zuverlässig für chirurgische Implantationen anzuwenden ist.

Diese Aufgabe wird gelöst durch ein strangförmiges Implantat aus resorbierbarem Polymermaterial, das als im wesentlichen statistisches Copolymer aus L-Lactid und Glykolid gebildet ist, wobei L-Lactid und Glykolid in einer Zusammensetzung im Bereich von mehr als 80 Mol-% Lactid und weniger als 20 Mol-% Glykolid bis 95 Mol-% Lactid und 5 Mol-% Glykolid, insbesondere im Verhältnis von 90:10 vorhanden sind, das Polymermaterial in seiner Molekularstruktur nicht endverkappt ist, in textiler Konstruktion eine Zugfestigkeit von mehr als 200 N/mm² aufweist und die inhärente Viskosität des Polymermaterials des fertigen Implantats 0,7 bis 1,3 dl/g beträgt. Bevorzugt ist eine Zugfestigkeit von mehr als 250 N/mm², bezogen auf den Gesamtquerschnitt des Implantats. Da die Konstruktion in der Regel nur etwa 50 bis 70 % Polymermaterial im Querschnitt enthält, ist die Zugfestigkeit bezogen auf das Polymer der Konstruktion entsprechend höher. Die Zugfestigkeit kann im Bereich von 400 bis 500 N/mm² liegen.

Mit Vorteil kann sich das erfindungsgemäße Implantat durch eine Bruchdehnung von weniger als 30 %, insbesondere weniger als 20 % auszeichnen. Bevorzugt kann ein Implantatstrang gemäß der Erfindung eine Bruchdehnung von 15 bis 20 % aufweisen. Erfindungsgemäß sind Implantate bevorzugt, die eine geringe Dehnung in Kombination mit hoher Reißkraft aufweisen.

Aufgrund der Korrelation von Struktur und Polymereigenschaften ergeben sich hieraus besonders vorteilhafte physikalische und chemische Eigenschaften für das Implantat gemäß der Erfindung. Es besteht insbesondere ausschließlich aus Lactid- und Glykolidbestandteilen. Eine Endverkappung entfällt.

Zum Vorteil einer erfindungsgemäßen Anwendung kann das mittlere Molekulargewicht des resorbierbaren Polymermaterials mindestens 50000 Dalton betragen. Erfindungsgemäß kann das mittlere Molekulargewicht 100000 bis 500000, insbesondere 200000 Dalton betragen. Ein solcher mittlerer Molekulargewichtswert kann auch durch Mischung von Polymeranteilen unterschiedlichen Molekulargewichts erreicht sein.

Die Einstellung des gewünschten Molekulargewichts kann während des Polymerisationsvorgangs nach den Fachleuten bekannten Verfahrenstechniken erfolgen. Bei langdauernder Polymerisationsreaktion kann sich durch Ausbildung einer Gleichgewichtsreaktion die Viskosität des erhaltenen Polymerprodukts wieder verringern. Eine Überprüfung des erreichten Polymerisationsgrades und Molekulargewichts kann durch Probennahme und Messung der Viskosität erfolgen. Viskositätsmessungen in Chloroform bei 25 °C in 0,1 %iger Lösung.

Mit Vorteil kann das Polymermaterial im fertigen Implantat einen Restmonomerengehalt von weniger als 1 Gew.-%, insbesondere weniger als 0,5 Gew.-%, bezogen auf das gesamte Polymer aufweisen. Das vorhandene Monomer kann Lactidmonomer, Glykolidmonomer oder eine Mischung von beiden sein. Der niedrige Gehalt an nicht umgesetztem Monomer kann das Abbauverhalten des resorbierbaren Polymers in für die Erfindung vorteilhafter Weise beeinflussen. Durch Steuerung des Gehalts an nicht polymerisiertem Monomer kann das Abbauprofil beeinflußt werden. Die Einstellung des Monomerenrestgehalts kann während des Polymerisationsvorgangs nach den Fachleuten bekannten Verfahrenstechniken erfolgen.

Die inhärente Viskosität des Polymermaterials liegt im fertigen Implantat erfindungsgemäß im Bereich von 0,7 bis 1,3 dl/g.

Beispielsweise kann bevorzugt die inhärente Viskosität eines aus dem erfindungsgemäßen Polymermaterial ersponnenen Rohfadens bei 0,9 bis 1,2 dl/g liegen. Ferner kann bevorzugt die inhärente Viskosität eines aus dem erfindungsgemäßen Polymermaterial hergestellten textilen Strangs bei 0,9 bis 1,1 dl/g liegen.

Die chemische Zusammensetzung und Molekularstruktur der erfindungsgemäßen Copolymere wirkt sich in Kombination mit der Konstruktion des Implantats auch in vorteilhafter Weise auf die Eigenschaften daraus hergestellter Produkte aus. Als Beispiele sind günstige mechanische Eigenschaften wie gute Flexibilität, beispielsweise geringe Biegesteifigkeit, gutes Modulverhalten und gute Verknotungseigenschaften zu nennen, wie sie insbesondere bei Anwendungen im medizinischen Bereich, etwa bei chirurgischen Implantaten, erwünscht sind.

Der Abbau des erfindungsgemäßen Implantats erfolgt im Körper eines Tieres oder eines Menschen, dem sie während eines chirurgischen Eingriffs implantiert wurde, durch Hydrolysevorgänge. An der Umsetzung sind unter physiologischen Bedingungen Körper- und Gewebeflüssigkeiten beteiligt. Der Zersetzungsvorgang kann auf hydrolytischem und/oder enzymatischem Wege erfolgen, wobei die Polymerkette in kleinere und leichter lösliche Fragmente gespalten wird. Die Abbauprodukte werden durch das Stoffwechselsystem abtransportiert und wie andere Stoffwechselschlacken aus dem Organismus ausgeschieden. Für eine gute Verträglichkeit des resorbierbaren Implantatmaterials beim Patienten ist es wichtig, daß sich während des Abbauvorganges keine schädlichen Metaboliten bilden oder anreichern. Die erfindungsgemäßen unverkappten Lactid-Glykolidpolymere zeichnen sich insbesondere dadurch aus, daß bei ihrer Zersetzung in vivo keine toxischen Zerfallsprodukte gebildet werden. Bei experimentellen Untersuchungen an den erfindungsgemäßen strangförmigen Implantaten aus resorbierbarem Polymer wurde gefunden, daß der Abbau in vitro ungefähr gleich dem Abbau in vivo verläuft.

Das erfindungsgemäße Implantat kann sich mit Vorteil dadurch auszeichnen, daß die Halbwertszeit seiner Festigkeit in vitro und in vivo 8 bis 16 Wochen, insbesondere 10 bis 14 Wochen beträgt. Dies bedeutet, daß nach Ablauf von 10 bis 14 Wochen die verbleibende Festigkeit noch die Hälfte des ursprünglichen Festigkeitswertes aufweist. Innerhalb dieses Zeitraums kann sich das natürliche körpereigene Gewebe so weit neubilden, daß es die Festigkeitsfunktionen wieder übernehmen kann. Auf diese Weise kommt es allmählich zu einer gleitenden Übernahme der vom Implantat erfüllten Funktion durch das natürliche Körpergewebe, bis schließlich das Implantatmaterial vollständig resorbiert und aus dem Körper entfernt ist. Bevorzugt kann sich das erfindungsgemäße Implantat dadurch auszeichnen, daß seine Resorptionszeit in vivo 6 bis 18 Monate beträgt. Mit Vorteil kann die Resorptionszeit des Implantats insbesondere 9 bis 12 Monate betragen.

Das für den erfindungsgemäßen Implantatstrang einzusetzende Copolymer aus L-Lactid und Glykolid kann durch statistische Copolymerisation der Ausgangsmonomeren erhalten werden. Der Monomerenmischung kann ein geeigneter Katalysator, z. B. Zinnoctoat, in der üblichen erforderlichen Menge zugesetzt werden. Die Umsetzung wird als Schmelzpolymerisation bei Temperaturen über 150 °C in einem geeigneten Reaktor durchgeführt, der heizbar und mit einer Rührvorrichtung versehen ist. Insbesondere ist dieser Polymerisationsreaktor so konzipiert, daß die entstehenden hochviskosen Schmelzen homogenisiert, die geforderten Temperaturbereiche eingehalten werden können und das Rohpolymerisat aus dem Reaktor weitgehend vollständig abgelassen werden kann.

Die Lactid-Glykolid-Copolymeren können durch übliche Schmelzspinnprozesse zu Filamenten extrudiert werden. Das Verspinnen der Schmelze kann mit Vorteil bei Temperaturen unter 210 °C, insbesondere bei Temperaturen im Bereich von 160 bis 200 °C vorgenommen werden.

Mit Vorteil kann das Copolymer mit einer inhärenten Viskosität von 1,2 bis 2,3 dl/g, insbesondere 1,4 bis 2,0 dl/g extrudiert werden.

Um die erforderlichen mechanischen Eigenschaften zu erhalten, kann das extrudierte Filament zur Orientierung der Molekülketten verstreckt werden. Der verfestigte Spinnfaden kann entweder direkt oder nach Aufspulen in einem gesonderten Schritt nach den Fachleuten bekannten Methoden verstreckt werden.

Um einen andauernden Erhalt der Orientierung, der mechanischen Eigenschaften sowie der Dimensionsstabilität der Filamente des fertigen Implantats zu sichern, kann das verstreckte Polymermaterial durch Tempern fixiert werden. Das Fixieren erfolgt bei Temperaturen im Bereich zwischen 50 °C und 150 °C, bevorzugt bei 70 bis 130 °C. Die Dauer des Thermofixierverfahrens liegt zwischen einer und 72 Stunden. Das Tempern kann mit oder ohne Schrumpfen des Filaments vorgenommen werden. Besonders bevorzugt ist es, Verstrecken und Thermofixieren unmittelbar anschließend an die Extrusion, insbesondere in einem kombinierten Verfahren durchzuführen. Mit Vorteil kann dazu eine entsprechende Apparatur von kombinierten geeigneten Vorrichtungen verwendet werden. In einer bevorzugten Ausführungsform der Erfindung können monofile oder multifile Produkte aus dem Copolymer zur Erzielung einer Dimensionsstabilität über einen Zeitraum von 20 bis 50 Stunden, mit oder ohne Schrumpf, einer Temperatur von 50 bis 150 °C ausgesetzt werden.

In einer Ausführungsform des erfindungsgemäßen Verfahrens kann das Copolymer aus der Schmelze zu Filamenten versponnen werden, wobei die Filamente bei 80 bis 120 °C mit einem Verstreckungsverhältnis von 1 : 2 bis 1 : 10 verstreckt und getempert werden.

Filamentgarne aus dem resorbierbaren Polymermaterial gemäß der Erfindung können nach den Fachleuten bekannten textilen Techniken zu strangförmigen Implantaten verarbeitet werden.

In einer Ausführungsform der Erfindung kann das Implantat in Form eines flachen Bandes ausgebildet sein.

In einer anderen Ausführungsform der Erfindung kann das Implantat in Form einer Kordel mit im wesentlichen rundem Querschnitt ausgebildet sein.

In einer weiteren Ausführungsform der Erfindung kann das Implantat in Form eines Schlauches oder eines flachen Schlauches ausgebildet sein.

In einer bevorzugten Ausführungsform kann sich das erfindungsgemäße Implantat dadurch auszeichnen, daß es als Verbundstruktur vom Typ Seele-Mantel ausgebildet ist.

Mit Vorteil kann beim Implantat gemäß der Erfindung die Seele aus einem Garn ausgebildet sein. In einer bevorzugten Ausführungsform kann die Seele aus mehreren Garnen ausgebildet sein. Erfindungsgemäß kann ein solches Garn aus 3 bis 300 Einzelfilamenten, insbesondere 7 bis 200 Einzelfilamenten ausgebildet sein.

In einer anderen Ausführungsform kann das Implantat gemäß der Erfindung als Nähfaden ausgebildet sein.

Die Herstellung des oder der für die Seele des erfindungsgemäßen Implantats zu verwendenden Garne kann nach den Fachleuten bekannten textilen Verfahren zur Garnherstellung erfolgen. Mit Vorteil können die Fasern im Garn einen leichten Drall aufweisen. Erfindungsgemäß bevorzugt kann das Garn gefacht und/oder gezwirnt sein.

Im Implantat gemäß der Erfindung können Einzelfasern eine Dicke von mehr als 1 bis 100 µm aufweisen, inbesondere von 10 bis 20 µm. Letzteres entspricht einer Faserstärke von 1 bis 4 dtex.

In Weiterbildung können beim erfindungsgemäßen Implantat in einer Verbundstruktur Einzelfasern verschiedener Dicken vorhanden sein. Insbesondere können Fasern im innen gelegenen Bereich der Implantatkonstruktion dicker sein als Fasern im äußeren Bereich der Implantatkonstruktion.

In einer besonderen Ausführungsform der Erfindung können sogenannte Stehfäden in der Verbundstruktur des Implantats eingebracht sein.

Mit Vorteil können in einer Ausführungsform der Verbundstruktur die Polymermaterialien einzelner textiler Komponenten ähnliche physikalische und mechanische Eigenschaften und ein ähnliches Resorptionsverhalten aufweisen. Auf diese Weise ist eine möglichst gleichmäßige Aufnahme und Verteilung der auftretenden Kräfte nach Einsetzen des Implantats im Patienten möglich. Eine möglichst gleichmäßige Resorption erreicht eine gleichmäßigen Festigkeitsabnahme des erfindungsgemäßen Implantats, so daß keine Schwachstellen im teilweise resorbierten Implantat entstehen, die den Heilungsverlauf nachteilig beinflussen könnten.

In einer anderen Ausführungsform der Verbundstruktur, bei der ein selektiver Abbau bestimmter Teilstrukturen vorgesehen ist, können die Polymermaterialien einzelner textiler Komponenten ein unterschiedliches Resorptionsverhalten aufweisen. Unabhängig davon besteht das erfindungsgemäße Implantat aus vollständig resorbierbaren Bestandteilen.

Wie oben bereits erwähnt, können die Eigenschaften, insbesondere die mechanischen Eigenschaften, der strangförmigen Implantate durch geeignete Kombination von chemischer Zusammensetzung und strukturellem Aufbau der Implantate günstig beeinflußt werden. Für die hohe Zugfestigkeit und die geringe Bruchdehnung sind Strukturen bevorzugt, bei denen eine möglichst große Anzahl an Fasern oder Garnen einen möglichst geringen Winkel zur Längsrichtung des strangförmigen Implantats einnehmen, und zwar nicht nur im Mittel, sondern auch an einzelnen Stellen, die potentielle Bruchstellen sein können, wenn sie starke Biegungen aufweisen.

Das strangförmige Implant gemäß der vorliegenden Erfindung kann in einer Ausführungsform als gewebte Textilkonstruktion ausgebildet sein. Die Kettfäden übernehmen hierbei eine tragende Funktion und sind von Bedeutung für eine hohe Festigkeit.

In einer anderen Ausführungsform der Erfindung kann das strangförmige Implantat als geflochtene Konstruktion ausgebildet sein. Mit besonderem Vorteil kann die erfindungsgemäße Strangkonstruktion als Spiralgeflecht ausgebildet sein. Bevorzugt kann ein Spiralgeflecht mit einem geringen Flechtwinkel von 5 bis 30 ° (Winkelgrad), und 10 bis 40 Überschneidungen pro franz. Zoll (27 mm), bevorzugt 25 bis 35 Überschneidungen pro franz. Zoll vorgesehen sein.

Die Geflechtstruktur spielt für die Höhe der Reißfestigkeit des Stranggebildes eine Rolle. Bei zu starkem Flechtwinkel weisen die strangförmigen Produkte eine geringe Reißkraft auf. Ebenso ist die Bindung der Fäden in der textilen Konstruktion von Bedeutung. Bevorzugt sind bei einem Schlauchgeflecht 2 über 2 Fäden, gute Werte sind auch mit 1 über 1 Fäden zu erzielen, jedoch ergeben Bindungen mit 3 über 3 Fäden eine zu lose Konstruktion.

In einer Ausführungsform einer Flechtkonstruktion für das erfindungsgemäße Implantat kann ein Geflecht um eine Seele angeordnet sein. Als Material für die Seele kann je nach gewünschten Eigenschaften eine textile Konstruktion aus einem Garn, mehreren Garnen oder ein Gebilde aus Einzelfilamenten gewählt werden, wie sie oben beschrieben sind.

Mit Vorteil zeichnen sich erfindungsgemäße strangförmige Implantate durch eine geringe Biegesteifigkeit aus, was die Handhabung bei medizinischen Anwendungen erleichtert und das Verknotungsverhalten verbessert.

Die Eigenschaften der für Seele und Mantel zu verwendenden Materialien können so ausgewählt werden, daß sich eine optimale Verbundstruktur ergibt. Ebenso können die textilen Techniken zur Ausbildung von Seele und Mantel so ausgewählt werden, daß sich eine optimale Verbundstruktur ergibt. Mit Vorteil können die textilen Konstruktionstechniken für Seele und Mantel so aufeinander abgestimmt sein, daß sich im wesentlichen gleiche Verhaltenseigenschaften, beispielsweise Dehnung, für Seele und Mantel ergeben. Bei ungleichem Verhalten von Seele und Mantel kann es im Falle von Beanspruchung zu übermäßiger Belastung der schwächeren Komponente mit dem Risiko eines vorzeitigen Versagens kommen.

So kann beispielsweise bei einer Ausführungsform mit gefachter Seele und geflochtenem Mantel die Seele relativ dick sein, damit sie im wesentlichen die gesamte Last tragen kann. Bei Implantaten in Form einer Kordel mit im wesentlichen rundem Querschnitt können zwei Geflechtkonstruktionen mit gutem Zusammenhalt von Seele und Mantel vorgesehen sein.

Das erfindungsgemäße Implantat kann auch als dreidimensionale Geflechtkonstruktion ausgebildet sein, bei der der Verlauf der Fäden nicht nur in einer Ebene oder einer Mantelfläche, sondern in allen drei Dimensionen, also durch den geamten Geflechtskörper erfolgt.

In einer bevorzugten Ausführungsform eines geflochtenen strangförmigen Implantats kann bei einem Geflecht in Seele-Mantel-Konstruktion die Flechtung in die Seele hineinverlaufen. Eine solche Einflechtung erhöht den Verbund zwischen Seele und Mantel in vorteilhafter Weise. Die Einflechtung kann in der Weise erfolgen, daß das Mantelgeflecht durch die Seele hindurchgreift, so daß die Seele nicht mehr als getrennte Komponente erkennbar ist. Es ergibt sich ein sogenanntes 3D-Geflecht oder dreidimensionales Geflecht.

In einer weiteren Ausführungsform der Erfindung kann das Implantat als schlauchförmiges Geflecht mit Stehfäden ausgebildet sein. Die Stehfäden können in vorteilhafterweise zur Festigkeit und den mechanischen Eigenschaften beitragen.

In noch einer anderen Ausführungsform der Erfindung kann das Implantat als gewirkte Konstruktion ausgebildet sein. Eine Wirkware findet mit Vorteil bei schlauchförmigen Implantaten Anwendung.

Bei Implantaten, die erfindungsgemäß als Verbundstrukturen ausgebildet sind, kann der Verbund bevorzugt durch textile Verbindungstechniken ausgebildet sein. So können beispielsweise, wie oben schon ausgeführt wurde, bei geflochtenen Implantaten die Fäden von Seele und Mantel miteinander verflochten sein.

Ein erfindungsgemäßes Verfahren zur Herstellung eines strangförmigen Implantats ist dadurch gekennzeichnet, daß resorbierbares Polymermaterial, das als im wesentlichen statistisches Copolymer aus L-Lactid und Glykolid gebildet ist, wobei L-Lactid und Glykolid in einer Zusammensetzung im Bereich von mehr als 80 Mol-% Lactid und weniger als 20 Mol-% Glykolid bis 95 Mol-% Lactid und 5 Mol-% Glykolid, insbesondere im Verhältnis von 90:10 vorhanden sind, in Form von Fasern nach textilen Verarbeitungstechniken zu einem strangförmigen textilen Gebilde geformt wird, wobei der Strang eine Zugfestigkeit von mehr als 200 N/mm², insbesondere mehr als 250 N/mm² aufweist, bezogen auf den Gesamtquerschnitt des Geflechts.

Verfahren zur Herstellung des erfindungsgemäßen Polymermaterials wurden oben angegeben. Bevorzugt kann das Copolymer aus der Schmelze zu Filamenten versponnen werden, wobei die Filamente bei 80 bis 120 °C mit einem Verstreckungsverhältnis von 1 : 2 bis 1 : 10 verstreckt und getempert werden können.

Die Polymere und die daraus hergestellten medizinischen Produkte gemäß der vorliegenden Erfindung können gefärbt oder ungefärbt sein. Zum Einfärben können für resorbierbare medizinische Produkte von der amerikanischen FDA (Food and Drug Administration) zugelassene Farbstoffe verwendet werden wie beispielsweise D&C Green Nr. 6, D&C Violet Nr. 2 und andere.

Erfindungsgemäße Implantatstränge können nach üblichen Methoden zu für chirurgische Anwendungen geeignetem Implantatmaterial verarbeitet, z. B. auf geeignete Längen zugeschnitten werden. Um ein Auflösen der textilen Konstruktion am Ende des Strangs zu verhindern, können die Enden der Implantatstränge in geeigneter Weise gesichert werden. Beispielsweise können die Enden abgeschmolzen, vernäht, verklebt oder ultraschallgeschweißt sein. In einer Ausführungsform kann an einem oder beiden Enden des strangförmigen Implantats ein chirurgisches Hilfsmittel wie etwa eine chirurgische Nähnadel oder ein Durchzieher vorgesehen sein.

Die wünschenswerten Eigenschaften des erfindungsgemäßen Implantats ergeben sich in vorteilhafter Weise aus der Kombination der Eigenschaften des resorbierbaren Copolymermaterials und der textilen Konstruktion des strangförmigen Gebildes. Bei histologischen Untersuchungen zeigte sich keine Reizung des Gewebes bei Implantation des erfindungsgemäßen Strangs im Tierversuch.

Zur Verwendung in der Medizin wird das erfindungsgemäße Polymermaterial insbesondere in geeigneter Weise sterilisiert. Ein zweckmäßiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfaßt die Behandlung mit γ-Strahlung. Bevorzugt kann eine Sterilisierung des erfindungsgemäßen Polymermaterials für medizinische Produkte unter Verwendung von Ethylenoxid vorgenommen werden.

Mit Vorteil kann das erfindungsgemäß hergestellte chirurgische Implantatmaterial in zweckmäßiger Länge zugeschnitten gebrauchsfertig in geeigneter Weise steril verpackt vorliegen. In einer bevorzugten Ausführungsform können die erfindungsgemäßen Stränge in für spezielle Anwendungen geeigneter Form mit chirurgischen Hilfsmitteln wie transossären Durchziehern versehen vorliegen.

Wegen der hydrolytischen Zersetzbarkeit des erfindungsgemäßen Polymermaterials sind die medizinischen Produkte bei ihrer Lagerung vor Feuchtigkeit und erhöhten Temperaturen zu schützen, damit die Festigkeitseigenschaften bis zur Verwendung voll erhalten bleiben. Mit Vorteil können gemäß der Erfindung hergestellte medizinische Stränge in gebrauchsbereitem Zustand getrocknet und in geeigneter Weise verpackt werden. Zweckmäßigerweise kann dies durch eine vor Feuchtigkeit schützende Verpackung geschehen, insbesondere einer Verpackung aus feuchtigkeitsundurchlässigem Folienmaterial, bevorzugt einer Vakuumverpackung. Ferner durch Auswahl eines trockenen kühlen Lagerortes.

Als Anwendungen für das erfindungsgemäße strangförmige Implantat sind Ersatztransplantationen, Prothesen sowie Augmentationstransplantationen und Augmentationsplastiken insbesondere im Bereich des Bewegungsapparates bei Menschen oder Tieren zu nennen. Beispiele für solche chirurgischen Anwendungen sind Behandlungen von Schäden an Bändern, Sehnen oder Knochen bedingt durch Verletzung oder Erkrankung. Solche Implantate können für den Patienten erhebliche Fortschritte in der Mobilität bringen. Als typische Anwendungen für das erfindungsgemäße Implantat sind beispielsweise Augmentation des Kreuzbandes, Fixierungen am Knochen und Befestigung von Weichgewebe zu nennen.

Das implantierte Polymermaterial kann die bei Bewegung des Patienten ausgeübten Kräfte aufnehmen und von der verletzten Körperstruktur fernhalten. So kann dieser Bereich unbelastet ausheilen, während das resorbierbare Polymer nach und nach abgebaut wird und der natürlichen Struktur ihre Funktion zurückgibt. Auf diese Weise wird eine frühe Mobilisierung des Patienten nach dem Eingriff möglich, was die bekannten durch postoperative Ruhigstellung bedingten Beeinträchtigungen des Bewegungsapparates und des Wohlbefindens vermeidet.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

### Beispiel 1

### Polymerisation

Ein 10 l Doppelmantelreaktor mit Paddelrührer, Rührmotor und Gaseinlaß wird mit 5400 g (37,5 Mol) L-Lactid und 600 g (5,17 Mol) Glycolid befüllt. Nach Zugabe von 1,2 g (0,0029 Mol) Zinnoctoat (2-Ethylhexansäure-Zinn(II)-Salz) gelöst in Diethylether wird der Reaktor verschlossen, evakuiert und die Mischung unter Rühren auf 80 °C erwärmt. Nach 1 bis 2 Stunden wird der Reaktor mit Argon unter 1 bis 2 bar Druck gesetzt, die Temperatur auf 200 °C erhöht und unter Rühren das Reaktionsgemisch ca. 2 bis 3 Stunden belassen. Anschließend wird die Reaktionsmasse abgelassen und granuliert.

Der Schmelzpunkt des granulierten Polymers liegt bei 152 °C (± 2 °C), der Glasumwandlungspunkt bei 51 °C (± 2 °C). Die inhärente Viskosität beträgt 1,6 ± 0,2 dl/g (0,1 %ige Lösung in Chloroform).

### Extrusion und Verstreckung

Das getrocknete Polymer (Restfeuchtegehalt < 0,01 %) wird mittels eines Schneckenextruders unter trockenem Stickstoffstrom zu einem 20 bzw. 40 Filamente umfassenden Fadenstrang extrudiert. Der Druck vor der Spinnpumpe beträgt 50 bar, der Druck zwischen Spinnpumpe und Düse liegt abhängig von der Ausgangsviskosität des Polymer, der Siebeinlage und dem Durchsatz an der Spinnpumpe im Bereich 100 bis 200 bar. Die Temperatur der Spinnplatte wird bei 190 °C gehalten. Der Fadenstrang wird mit einer Geschwindigkeit von 500 bis 1000 m/min von einer Aufnahmespule aufgenommen.

Der so erhaltene Multifilamentstrang wird durch Verstrecken über eine Schiene um den Faktor 2 bis 5 orientiert. Der Faktor ist abhängig von der Abzugsgeschwindigkeit der Aufnahmespule. Die Zugfestigkeit des Multifilamentgarnes beträgt nach dieser Wärmebehandlung 40 bis 45 cN/tex. Der Titer des Garnes mit 20 Filamenten liegt im Bereich 40 bis 60 dtex, der mit 40 Filamenten liegt im Bereich 80 bis 120 dtex. Die inhärente Viskosität beträgt 1,1 dl/g (0,1 %ige Lösung in chloroform).

### Strangkonstruktion

Zur Herstellung einer Kordel mit einem Durchmesser von 2,0 mm wurden folgende Flechtparameter eingestellt, wobei bei den Titerangaben zu berückichtigen ist, daß es sich um gefachte Seelen- und Mantelkonstruktionen handelt.

### Thermische Nachbehandlung (Posttreatment-Verfahren)

Um das Degradationsverhalten der Kordel zu verbessern, d. h. Halbwertszeiten des mechanischen Abbaus zu erhöhen, ist es erforderlich, in einem thermischen Nachbehandlungsprozess den Monomergehalt in der Polymermatrix deutlich zu reduzieren.

Hierbei wird die fertige Strangkonstruktion auf eine zylindrische Stahlrolle unter einer Spannung von 10 N aufgebracht und 40 Stunden bei 90 °C und einem Vakuum von < 1 mbar nachbehandelt. Der gemessene Monomergehalt an Lactid liegt nach dem Posttreatment-Prozess unter 0,3 %.

### Beispiel 2

**Schlauchgeflecht bestehend aus Seele und Mantel**

| | |
|---|---|
| Seele: | 20 Klöppel x 750 dtex |
| Mantel: | 24 Klöppel x 232 dtex |
| Flechten/Zoll: | Mantel: 60, Seele: gefacht |
| Titer: | 2276 tex |
| Durchmesser: | 2,04 mm |
| Höchstzugkraft: | 713 N |
| Reißfestigkeit: | 226 N/mm² |
| Dehnung bei Höchstzugkraft: | 18,8 % |
| Halbwertszeit in vitro: | ca. 77 Tage |

### Beispiel 3

**Schlauchgeflecht, Mantelgeflecht**

| Seele: | |
|---|---|
| Mantel: | 24 Klöppel x 840 dtex |
| Flechten/Zoll: | Mantel: 8 |
| Titer: | 2142 tex |
| Durchmesser: | 1,85 mm |
| Höchstzugkraft: | 680 N |
| Reißfestigkeit: | 225 N/mm² |
| Dehnung bei Höchstzugkraft: | 19,5 % |
| Halbwertszeit in vitro: | ca. 80 Tage |

### Beispiel 4

**Spiralgeflecht**

| | |
|---|---|
| Seele: | 9 Klöppel x 350 dtex |
| Mantel: | 20 Klöppel x 1050 dtex |
| Flechten/Zoll: | Mantel: 15, Seele: 26 |
| Titer: | 2539 tex |
| Durchmesser: | 2,22 mm |
| Höchstzugkraft: | 660 N |
| Reißfestigkeit: | 205 N/mm² |
| Dehnung bei Höchstzugkraft: | 17,3 % |
| Halbwertszeit in vitro: | ca. 85 Tage |

### Beispiel 5

**Schlauchgeflecht**

| | |
|---|---|
| Seele: | 8 Klöppel x 1050 dtex |
| Mantel: | 16 Klöppel x 897 dtex |
| Flechten/Zoll: | Mantel: 24, Seele: 16 |
| Titer: | 2402 tex |
| Durchmesser: | 1,98 mm |
| Höchstzugkraft: | 731 N |
| Reißfestigkeit: | 232 N/mm² |
| Dehnung bei Höchstzugkraft: | 16,9 % |
| Halbwertszeit in vitro: | ca. 80 Tage |

## Patentansprüche

1. Strangförmiges Implantat ausgebildet aus resorbierbarem Polymermaterial, das als im Wesentlichen statistisches Copolymer aus L-Lactid und Glykolid gebildet ist, wobei L-Lactid und Glykolid in einer Zusammensetzung im Bereich von mehr als 80 Mol-% Lactid und weniger als 20 Mol-% Glykolid bis 95 Mol-% Lactid und 5 Mol-% Glykolid, insbesondere im Verhältnis von 90:10 vorhanden sind, das Polymermaterial in seiner Molekularstruktur nicht endverkappt ist, und das in einer textilen Konstruktion aus Polymerfasergarn eine Zugfestigkeit von mehr als 200 N/mm² aufweist und die inhärente Viskosität des Polymermaterials des fertigen Implantats 0,7 bis 1,3 dl/g beträgt.

2. Implantat nach Anspruch 1, **dadurch gekennzeichnet, dass** es als strangförmiges textiles Gebilde vorliegt, das insbesondere aus Filamentgarnen gebildet ist.

3. Implantat nach Anspruch 1 oder 2, **dadurch gekennzeichnet, dass** seine Bruchdehnung weniger als 30 %, insbesondere weniger als 20 % beträgt.

4. Implantat nach einem der vorhergehenden Ansprüche, **dadurch gekennzeichnet, dass** das Polymermaterial des fertigen Implantats einen Restmonomerengehalt von weniger als 1 Gew.-%, insbesondere weniger als 0,5 Gew.-%, bezogen auf das gesamte Polymer aufweist.

5. Implantat nach einem der vorhergehenden Ansprüche, **dadurch gekennzeichnet, dass** die inhärente Viskosität des Polymermaterials des fertigen Implantats 0,9 bis 1,2 dl/g beträgt.

6. Implantat nach einem der vorhergehenden Ansprüche, **dadurch gekennzeichnet, dass** die Halbwertszeit seiner Festigkeit in vitro und in vivo 8 bis 16 Wochen, insbesondere 10 bis 14 Wochen beträgt.

7. Implantat nach einem der vorhergehenden Ansprüche, **dadurch gekennzeichnet, dass** seine Resorptionszeit in vivo 6 bis 18 Monate, insbesondere 9 bis 12 Monate beträgt.

8. Implantat nach einem der vorhergehenden Ansprüche, **dadurch gekennzeichnet, dass** es als strangförmiges textiles Gebilde aus Polymerfasergarn gebildet biegeschlaff ist.

9. Implantat nach einem der vorhergehenden Ansprüche, **dadurch gekennzeichnet, dass** es in Form einer Kordel mit im Wesentlichen rundem Querschnitt ausgebildet ist.

10. Implantat nach einem der vorhergehenden Ansprüche, **dadurch gekennzeichnet, dass** das Garn aus 3 bis 300 Einzelfilamenten, insbesondere 7 bis 200 Einzelfilamenten ausgebildet ist.

11. Implantat nach einem der vorhergehenden Ansprüche, **dadurch gekennzeichnet, dass** das Garn gefacht und/oder gezwirnt ist.

12. Implantat nach einem der vorhergehenden Ansprüche, **dadurch gekennzeichnet, dass** Einzelfasern eine Dicke von 1 bis 100 µm, insbesondere von 10 bis 20 µm aufweisen.

13. Implantat nach einem der vorhergehenden Ansprüche, **dadurch gekennzeichnet, dass** in einer Verbundstruktur Einzelfasern verschiedener Dicken vorhanden sind, insbesondere Fasern im innen gelegenen Bereich dicker sind als außen.

14. Implantat nach einem der vorhergehenden Ansprüche, **dadurch gekennzeichnet, dass** in einer Verbundstruktur die Polymermaterialien einzelner textiler Komponenten ein unterschiedliches Resorptionsverhalten aufweisen.

15. Implantat nach einem der vorhergehenden Ansprüche, **dadurch gekennzeichnet, dass** es als gewebte Konstruktion, insbesondere mit den Kettfäden in Längsrichtung ausgebildet ist.

16. Implantat nach einem der vorhergehenden Ansprüche, **dadurch gekennzeichnet, dass** es als geflochtene Konstruktion ausgebildet ist.

17. Implantat nach einem der vorhergehenden Ansprüche, **dadurch gekennzeichnet, dass** es als Spiralgeflecht ausgebildet ist.

18. Implantat nach einem der vorhergehenden Ansprüche, **dadurch gekennzeichnet, dass** ein Spiralgeflecht mit einem geringen Flechtwinkel von 5 bis 30 Winkelgrad, und 10 bis 40 Überschneidungen pro 27 mm, bevorzugt 25 bis 35 Überschneidungen pro 27 mm ausgebildet ist.

19. Implantat nach einem der vorhergehenden Ansprüche, **dadurch gekennzeichnet, dass** bei Verbundstrukturen der Verbund durch textile Verbindungstechniken ausgebildet ist.

20. Verfahren zur Herstellung eines strangförmigen Implantats nach einem der Ansprüche 1-19, **dadurch gekennzeichnet, dass** resorbierbares Polymermaterial, das als im Wesentlichen statistisches Copolymer aus L-Lactid und Glykolid gebildet ist, wobei L-Lactid und Glykolid in einer Zusammensetzung im Bereich von mehr als 80 Mol-% Lactid und weniger als 20 Mol-% Glykolid bis 95 Mol-% Lactid und 5 Mol-% Glykolid, insbesondere im Verhältnis von 90:10 vorhanden sind, wobei das Polymermaterial in seiner Molekularstruktur nicht endverkappt ist, in Form von Fasern nach textilen Verarbeitungstechniken zu einem strangförmigen textilen Gebilde geformt wird, so dass der Strang eine Zugfestigkeit von mehr als 200 N/mm² aufweist.

21. Verfahren nach Anspruch 20, **dadurch gekennzeichnet, dass** das Copolymer aus der Schmelze zu Filamenten versponnen wird, wobei die Filamente bei 80 bis 120 °C mit einem Verstreckungsverhälntis von 1 : 2 bis 1 : 10 verstreckt und getempert werden.

22. Verfahren nach Anspruch 20 oder 21, **dadurch gekennzeichnet, dass** das Verspinnen der Schmelze bei Temperaturen unter 210 °C, insbesondere im Bereich von 160 bis 200 °C vorgenommen wird.

23. Verfahren nach einem der Ansprüche 20 bis 22, **dadurch gekennzeichnet, dass** das Copolymer mit einer inhärenten Viskosität von 1,2 bis 2,3 dl/g, insbesondere 1,4 bis 2,0 dl/g extrudiert wird.

24. Verwendung des strangförmigen Implantatsnach einem der Ansprüche 1 bis 19 in textiler Konstruktion aus resorbierbarem Polymermaterial, in der Herstellung von Prothesen und/oder Augmentationsplastiken in der Chirurgie.

## Claims

1. Strand-like implant of resorbable polymer material which is essentially formed as a random copolymer of L-lactide and glycolide, in which L-lactide and glycolide are present in a composition in the range of more than 80 mole % lactide and less than 20 mole % glycolide to 95 mole % lactide and 5 mole % glycolide, particularly in a ratio of 90:10, the polymer material is not end capped in its molecular structure, and which in a textile structure of polymeric fibrous yarn has a tensile strength of more than 200 N/mm², and the inherent viscosity of the polymer material of the finished implant is 0.7 to 1.3 dl/g.

2. Implant according to Claim 1, **characterized in that** it is present as a strand-like textile construction which is formed in particular from filament yarns.

3. Implant according to Claim 1 or 2, **characterized in that** its elongation at break is less than 30%, and in particular less than 20%.

4. Implant according to any one of the preceding claims, **characterized in that** the polymer material of the finished implant has a residual monomer content of less than 1 wt. %, and in particular less than 0.5 wt. %, based on the total polymer.

5. Implant according to any one of the preceding claims, **characterized in that** the inherent viscosity of the polymer material of the finished implant is 0.9 to 1.2 dl/g.

6. Implant according to any one of the preceding claims, **characterized in that** the half-life period of its strength in vitro and in vivo is 8 to 16 weeks, and in particular 10 to 14 weeks.

7. Implant according to any one of the preceding claims, **characterized in that** its in vivo resorption time is 6 to 18 months, and in particular 9 to 12 months.

8. Implant according to any one of the preceding claims, **characterized in that** as a strand-like textile construction formed from polymeric fibrous yarn it is bending slack.

9. Implant according to any one of the preceding claims, **characterized in that** it is formed in the shape of a cord with a substantially round cross-section.

10. Implant according to any one of the preceding claims, **characterized in that** the yarn is formed from 3 to 300 individual filaments, and in particular from 7 to 200 individual filaments.

11. Implant according to any one of the preceding claims, **characterized in that** the yarn is folded and/or plied.

12. Implant according to any one of the preceding claims, **characterized in that** individual fibres have a thickness of 1 to 100 µm, and in particular of 10 to 20 µm.

13. Implant according to any one of the preceding claims, **characterized in that** one composite construction comprises individual fibres of various thicknesses, in particular fibres in the inner area are thicker than on the outside.

14. Implant according to any one of the preceding claims, **characterized in that** in one composite construction the polymer materials of individual textile components have different resorption characteristics.

15. Implant according to any one of the preceding claims, **characterized in that** it is formed as a woven structure, in particular with the warp threads in the longitudinal direction.

16. Implant according to any one of the preceding claims, **characterized in that** it is formed as a braided structure.

17. Implant according to any one of the preceding claims, **characterized in that** it is formed as a spiral braid.

18. Implant according to any one of the preceding claims, **characterized in that** a spiral braid having a low braiding angle of 5 to 30 degrees of angle and 10 to 40 overlaps per 27 mm and preferably 25 to 35 overlaps per 27 mm is formed.

19. Implant according to any one of the preceding claims, **characterized in that** in composite constructions the composite is formed by textile joining techniques.

20. Process for producing a strand-like implant according to any one of Claims 1-19, **characterized in that** the resorbable polymer material which is essentially formed as a random copolymer of L-lactide and glycolide, in which L-lactide and glycolide are present in a composition in the range of more than 80 mole % lactide and less than 20 mole % glycolide to 95 mole % lactide and 5 mole % glycolide, particularly in a ratio of 90:10, the polymer material is not end capped in its molecular structure, and is in the form of fibres shaped by textile processing techniques into a strand-like textile construction, so that the strand has a tensile strength of more than 200 N/mm².

21. Process according to Claim 20, **characterized in that** the copolymer is spun from the melt into filaments, which are drawn and annealed at 80 to 120°C with a drawing ratio of 1:2 to 1:10.

22. Process according to Claim 20 or 21, **characterized in that** the spinning of the melt is effected at temperatures below 210°C, in particular in the range from 160 to 200°C.

23. Process according to any one of Claims 20 to 22, **characterized in that** the copolymer extruded has an inherent viscosity of 1.2 to 2.3 dl/g, and in particular 1.4 to 2.0 dl/g.

24. Use of the strand-like implant according to any one of Claims 1 to 19 in a textile structure of resorbable polymer material, in the manufacture of prostheses and/or augmentation transplants in surgery.

## Revendications

1. Implant en forme de ruban, constitué d'un matériau polymère résorbable qui est formé essentiellement d'un copolymère statistique de L-lactide et de glycolide qui comprend le L-lactide et le glycolide dans une composition dans la plage de plus de 80% en moles de lactide et de moins de 20% en moles de glycolide à 95% en moles de lactide et 5% en moles de glycolide, en particulier dans le rapport de 90:10, la structure moléculaire du matériau polymère n'ayant pas de terminaison chapeautée, et qui présente dans une structure textile de fils de fibres polymères une résistance en traction supérieure à 200 N/mm², la viscosité intrinsèque du matériau polymère de l'implant prêt à l'emploi étant comprise entre 0,7 et 1,3 dl/g.

2. Implant selon la revendication 1, **caractérisé en ce qu'**il se présente sous la forme d'un produit textile en forme de ruban formé, en particulier de fils multifilaments.

3. Implant selon la revendication 1 ou 2, **caractérisé en ce que** son allongement à la rupture est inférieur à 30%, en particulier inférieur à 20%.

4. Implant selon l'une quelconque des revendications précédentes, **caractérisé en ce que** le matériau polymère de l'implant prêt à l'emploi présente une teneur résiduelle en monomère inférieure à 1% en poids, en particulier inférieure à 0,5% en poids par rapport à la totalité du polymère.

5. Implant selon l'une quelconque des revendications précédentes, **caractérisé en ce que** la viscosité intrinsèque du matériau polymère de l'implant prêt à l'emploi est de 0,9 à 1,2 dl/g.

6. Implant selon l'une quelconque des revendications précédentes, **caractérisé en ce que** tant in vitro qu'in vivo, la demi-vie de sa résistance mécanique est de 8 à 16 semaines, en particulier de 10 à 14 semaines.

7. Implant selon l'une quelconque des revendications précédentes, **caractérisé en ce que** sa durée de résorption in vivo est de 6 à 18 moi, en particulier de 9 à 12 mois.

8. Implant selon l'une quelconque des revendications précédentes, **caractérisé en ce qu'**il présente la forme d'un produit textile en forme de ruban en fil de fibres polymères et **en ce qu'**il est mollement flexible.

9. Implant selon l'une quelconque des revendications précédentes, **caractérisé en ce qu'**il présente la forme d'un cordon de section transversale essentiellement ronde.

10. Implant selon l'une quelconque des revendications précédentes, **caractérisé en ce que** le fil est formé de 3 à 300 filaments individuels, en particulier de 7 à 200 filaments individuels.

11. Implant selon l'une quelconque des revendications précédentes, **caractérisé en ce que** le fil est assemblé et/ou torsadé.

12. Implant selon l'une quelconque des revendications précédentes, **caractérisé en ce que** les fibres individuelles présentent une épaisseur de 1 à 100 µm, en particulier de 10 à 20 µm.

13. Implant selon l'une quelconque des revendications précédentes, **caractérisé en ce que** dans une structure composite, on trouve des fibres individuelles de différentes épaisseurs, en particulier les fibres de la zone intérieure sont plus épaisses que celles de la zone extérieure.

14. Implant selon l'une quelconque des revendications précédentes, **caractérisé en ce que** dans une structure composite, les matériaux polymères des composants textiles individuels présentent différents comportements de résorption.

15. Implant selon l'une quelconque des revendications précédentes, **caractérisé en ce qu'**il est configuré sous la forme de structure tissée, en particulier avec les fils de chaîne dans la direction longitudinale.

16. Implant selon l'une quelconque des revendications précédentes, **caractérisé en ce qu'**il est configuré sous la forme d'une structure tressée.

17. Implant selon l'une quelconque des revendications précédentes, **caractérisé en ce qu'**il est configuré en treillis en spirale.

18. Implant selon l'une quelconque des revendications précédentes, **caractérisé en ce qu'**un treillis en spirale à faible angle de treillis de 5 à 30 degrés angulaires est configuré et présente de 10 à 40 croisements pour 27 mm, de préférence de 25 à 35 croisements pour 27 mm.

19. Implant selon l'une quelconque des revendications précédentes, **caractérisé en ce que** dans les structures composites, le composite est formé par des techniques de liaison textile.

20. Procédé de fabrication d'un implant en forme de ruban selon l'une quelconque des revendications 1 à 19, **caractérisé en ce qu'**un matériau polymère résorbable sous forme de fibres, formé essentiellement d'un copolymère statistique de L-lactide et de glycolide, le L-lactide et le glycolide ayant une composition dans la plage de plus de 80% en moles de lactide et de moins de 20% en moles de glycolide à 95% en moles de lactide et 5% en moles de glycolide, en particulier dans le rapport de 90:10, la structure moléculaire du matériau polymère n'ayant pas de terminaison chapeautée, est transformé par des techniques de traitement textile en un produit textile en forme de ruban de telle sorte que le ruban présente une résistance en traction supérieure à 200 N/mm².

21. Procédé selon la revendication 20, **caractérisé en ce que** le copolymère est filé en filaments à partir de l'état fondu, les filaments étant étirés entre 80 et 120°C à un rapport d'étirage de 1:2 à 1:10 et étant trempés.

22. Procédé selon la revendication 20 ou 21, **caractérisé en ce que** le filage à l'état fondu est réalisé à des températures inférieures à 210°C, en particulier comprises dans la plage de 160 à 200°C.

23. Procédé selon l'une quelconque des revendications 20 à 22, **caractérisé en ce que** le copolymère est extrudé à une viscosité intrinsèque de 1,2 à 2,3 dl/g et en particulier de 1,4 à 2,0 dl/g.

24. Utilisation de l'implant en forme de ruban selon l'une quelconque des revendications 1 à 19 dans une structure textile en matériau polymère résorbable, pour la réalisation de prothèses et/ou de plastiques de renforcement en chirurgie.
